(19) Europäisches Patentamt
European Patent Office
Office européen des brevets

(11) **EP 4 327 752 A2**

(12) **EUROPEAN PATENT APPLICATION**

(43) Date of publication:
**28.02.2024 Bulletin 2024/09**

(21) Application number: **23219509.9**

(22) Date of filing: **27.05.2020**

(51) International Patent Classification (IPC):
***A61B 8/08*** *(2006.01)*

(52) Cooperative Patent Classification (CPC):
**A61B 8/10; A61B 8/5223;** A61B 8/085

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.06.2019 GB 201908052**

(62) Document number(s) of the earlier application(s) in accordance with Art. 76 EPC:
**20737615.3 / 3 979 915**

(71) Applicant: **Nisonic AS**
**7462 Trondheim (NO)**

(72) Inventors:
• **SMISTAD, Erik**
**7030 Trondheim (NO)**

• **BREKKEN, Reidar**
**7026 Trondheim (NO)**
• **SELBEKK, Tormod**
**7043 Trondheim (NO)**

(74) Representative: **Dehns**
**St. Bride's House**
**10 Salisbury Square**
**London EC4Y 8JD (GB)**

Remarks:
This application was filed on 21-12-2023 as a divisional application to the application mentioned under INID code 62.

(54) **ALIGNMENT OF ULTRASOUND IMAGE**

(57) An apparatus for automated quantification of papilledema, the apparatus comprising: an ultrasound imaging device including an ultrasound probe; and a computer system configured to: use the ultrasound probe of the ultrasound imaging device to obtain images of a region including the eye circle 4 and optic nerve; using computer implemented image processing techniques, identify a principal direction 10 of the optic nerve and the curve of the eye circle 4; based on the intersection of the principal direction 10 and the curve of the eye circle 4, identify an area of the eye circle 4 relevant to papilledema; and using an image processing algorithm, find one or more parameter(s) relating to the shape and/or size of a formation 24 at the back of the eye 1 in the area of the eye circle 4 known to exhibit papilledema, and thereby quantify the papilledema.

EP 4 327 752 A2

Fig. 3

## Description

The present invention relates to a method and a system for alignment of an ultrasound image obtained with an ultrasound probe for obtaining images in relation to the optic nerve or associated anatomical structures.

It is desirable in various contexts to obtain images of anatomical structures relating to the eye. MRI can be used to obtain images in relation to the eye, as can various other techniques. Ultrasound imaging is widely used for imaging the eye.

In one example WO 2016/193168 describes methods for determining markers relating to intra-cranial pressure (ICP) based on assessment of the stiffness of the optic nerve sheath, with the use of images that may be obtained by ultrasound. The ultrasound images are used to assess the stiffness of the optic nerve sheath with reference to pulsatile dynamic movements of the optic nerve sheath, with a marker indicating possibly increased intracranial pressure being obtained by associating increased stiffness with increased intracranial pressure.

In other examples, ultrasound imaging is used for measurement of the diameter of the optic nerve or optic nerve sheath, and proposals have been made for automated image processing in order to obtain a diameter measurement or grading. One technique for measurement of the diameter of the optic nerve sheath is disclosed in US 8672851, which discusses automatic measurement via ultrasound images in order to provide a result indicating a healthy or at-risk patient based on the diameter. The method used for automation is said to include image processing to identify points around a diameter.

Viewed from a first aspect, the invention provides a method for alignment of an ultrasound image obtained with an ultrasound probe for obtaining images in relation to the eye, the method comprising:

placing the ultrasound probe in a suitable location for obtaining images of anatomical structures in the region of the eye and across a region of interest expected to include the optic nerve;
obtaining images of said region of interest using the ultrasound probe;
processing the images of said region of interest via computer implemented image processing techniques in order to identify boundary features in the images that are representative of the boundaries of at least one of the optic nerve and the optic nerve sheath;
using the identified boundary features of the imaged structure to determine a principal direction extending along the length of the optic nerve and the optic nerve sheath in the image;
identifying at least first and second points on the identified boundary features, wherein the first and second points are at different locations along the extent of the principal direction; and

rotating the image plane of the probe until the first and second points are aligned in the image and thereby determining a required orientation of the image plane of the probe for alignment of the ultrasound image with the principal direction.

The alignment of the image plane relative to the centre of the optic nerve at the region of interest may also involve extraction of cross sections of the boundary features of the optic nerve or the ONS. The alignment can be done in any direction relative to the displayed image, for example laterally or in the elevational direction. To obtain an image plane (2D real-time or an image plane extracted from a 3D image volume) that is slicing through the centre of the ON and ONS, the alignment in the direction perpendicular to the image plane may be done by finding the cross sections of the extracted boundary features in at least two different positions along the principal direction. The cross-section can be defined as two opposite positions on the extracted boundary features of the ON or ONS, being perpendicular to the same location on the axis represented by the principal direction. The image plane can be defined as aligned with the principal direction when the measured distance of the at least two extracted cross-sections is of identical or similar magnitude. The image plane can be defined to be aligned with the centre of the ONS and along the principal direction when the cross sections at the at least two positions are both at their maximum value.

With this method, the use of points at two depths identified based on alignment with the principal direction of the optic nerve sheath allows for accurate alignment of the image with the optic nerve sheath (and optic nerve). Effectively, the method involves alignment of the principal direction with the imaging axis of the ultrasound probe by rotating of the image plane of the probe. This then allows for further images to be obtained with a known and accurate alignment of the image, where the optic nerve sheath is "vertical" in relation to a probe image plane axis, which has benefits in various applications for assessment of the patient as discussed further below. As used herein, references to "vertical" are concerned with alignment with the main imaging axis of the ultrasound probe extending into the volume that is being imaged (axial direction), and "horizontal" refers to directions (and the plane) normal to that vertical (lateral direction) and the slice thickness refers to the direction being perpendicular to the image plane (elevational direction). It is noted that further directions relevant to alignment may also be determined, such that the principal direction referenced above may be considered as a first principal direction, and the method may then extend to determining a second principal direction and so on.

The principles of the method can be implemented for real-time 2D imaging, for three dimensional ultrasound volumes (acquired with or without the use of position tracking systems, or with mechanical movement of the array) or 4D ultrasound imaging. For example the method may be implemented with an ultrasound probe comprising a 2D array that obtains a 3D imaging volume. The method may also involve aligning the ultrasound image (or volume) in multiple directions. This may be achieved by repeating the method in two orthogonal directions in order to align the image, for example in the lateral and elevational direction.

The two points on the boundary features may for example be cross-sections of the boundary features. This approach also allows clear visualisation of the alignment of the image plane of the probe during rotating (and optionally translation), as discussed further below.

The rotating of the image plane of the probe could be done via any suitable technique, including via a suitable actuation mechanism for rotating the probe or via manual movement of the probe. It will be understood that the rotating of the image plane of the probe may include a rotation of the probe that turns the probe axis relative to an expected direction of the required principal direction (e.g. as based on direction the patient is facing). The rotating movement may be done without any twisting rotation of the probe, i.e. no rotation about the imaging axis of the probe. Although herein the method refers to rotating of the image plane of the probe, it will be understood that the resulting movement of the image plane of the probe could be equivalently described as tilting, i.e. a movement of the probe that causes the angle of the image plane to change. Additionally, it may not be the probe itself that is rotated, but a transducer array within the probe. Thus, it may be that the probe includes a suitable actuation mechanism in which case the method may involve using the actuation mechanism to rotate the probe, and hence rotate the image plane of the probe to align the first and second cross-sections. In this way the method may allow for a highly automated system in which the required alignment of the image is achieved via mechanised movement and with the principal direction of the optic nerve sheath being determined based on boundary features obtained by computer implemented image processing. This approach avoids the need for a highly trained person to analyse the ultrasound image data, and could be implemented via devices that do not require any manual input for handling of the probe.

An untrained user can hence be provided with accurate medical input based on subsequently obtained images. The method may also allow for electronic steering of the ultrasound beams generated by the ultrasound transducer array in order to achieve the required alignment. For example the first and second extracted cross sections may serve as inputs for electronic beam steering.

Alternatively, it may be that the probe is arranged to be handled manually so that the user is required to rotate the image plane of the probe in order to align the first and second points (e.g. first and second cross-sections). In this way the method makes innovative use of a "hybrid" automated and manual system. Whilst a manual movement is needed for the required alignment of the probe this can be done by relatively unskilled personnel whilst retaining a suitable degree of accuracy, allowing simplification of the probe device compared to a more automated arrangement. In common with the more automated approach described above, 'hybrid' manual-automatic approach avoids the need for a highly trained person to analyse the ultrasound image data. Thus, it enables accurate alignment with relatively straightforward manual input as well as the potential to use a simple (and cheap) ultrasound probe device. It will be understood that this can provide significant advantages in terms of ease of access to the benefits that are available from subsequent use of the aligned images.

In a further alternative, the alignment of the image relative to the defined cross sections can also be done by electronic steering of the beams of the transducer array. Thus, the image plane is rotated via an electronic beam steering technique rather than by a physical movement of the probe or of the array.

These benefits could arise in various contexts (for either the actuation mechanism, manual rotation of the probe, or beam steering), such as:

- Using the accurate alignment with the optic nerve sheath to enable capturing of accurate optic nerve sheath diameter measurements, optionally including automated assessment of the diameter.

- Automated measurement and assessment of the diameter of the optic nerve sheath with an indication of a healthy or at risk patient based on the diameter as in US 8672851.

- Processing of the subsequently obtained images in relation to the pulsatile dynamics and stiffness measure as proposed in WO 2016/193168, with the accuracy of alignment of the images allowing for more accurate automated processing if needed.

- Analysis of other parameters or conditions known to have an impact visible in ultrasound images, again with the accuracy of alignment of the images allowing for more accurate automated processing. An example of this is quantification of papilledema.

It is preferred for the method to include alignment of the centre of the ultrasound image with the principal direction. This can be checked by ensuring that the two points are aligned with each other whilst also being centrally placed within the ultrasound image from the probe. If necessary, the method may include a translation of the probe so as to move the centre of the ultrasound image for better alignment, i.e. a transverse movement of the probe relative to the principal direction. With an accurate initial placement of the probe in a suitable location relative to the eye there may not be any need for this movement. The placement of the probe in a suitable location may be achieved in most instances by reference to the patient's face and the shape/position of the eye. In some examples, the probe is provided with physical guide features to promote placement of the probe in a suitable location.

In the case of an actuation mechanism for rotating the image plane of the probe the method may include, after alignment of the two points, determining the location of the aligned points relative to the centre of the ultrasound image; and, if required, translating the probe to move the centre of the ultrasound image for better alignment with the aligned points.

The method may include providing feedback to the user in relation to the degree of alignment of the two points, and optionally also in relation to the relative position of the centre of the ultrasound image. This is of particular use in the case where the probe is arranged to be manually rotated, since it provides a convenient way to guide the user during the manual rotation. In one example the method includes using a visual display with representations of the two points (for example, representations of two cross-sections). This can enable the user to manually rotate the ultrasound probe into the best position by aligning the representations of the two points. The visual display may also include a representation of the centre of the ultrasound images, allowing the user to also manually translate the ultrasound probe to align the points with the centre of the ultrasound image, thus ensuring that the principal direction is horizontally positioned in the centre whilst also being vertically aligned with the probe axis. The visual display may include a 3D graphical representation of an imaging volume, for example a 3D imaging volume from an ultrasound probe with a 2D array, or a 3D imaging volume reconstructed from multiple 2D image planes.

The visual display may, for example, use two circles to represent two cross-sections and a further circle or another marking, such as a line, to represent the centre of the ultrasound image. Different colours may be used to allow the different features to be readily distinguished.

It should be noted that whilst the discussion above generally refers to the use of two points, the underlying image processing systems may identify more than two points and in fact may make use of large numbers of points to ensure accuracy in identifying the extent of the boundary features. Two points are the minimum needed to ensure alignment with the direction along the length of the optic nerve sheath, as well as providing a convenient way to visualise the proposed alignment process.

The two points are at different positions along the principal direction. They may be spaced apart by at least 1 mm or by at least 2 mm. In one example the two cross-sections are spaced apart by about 3 mm. They may be cross-sections at about 3mm and 6 mm depths below the eye circle. The method may include identifying the eye anatomy, in particular the scleral surface forming a curved line in the ultrasound images, with such a curved boundary feature hereafter referred to as the eye circle (within the ultrasound images) and thereby determining a distance along the principal direction from the eye circle. The eye and/or the eye circle may be identified during a segmentation of an ultrasound image that also identifies the boundary feature, i.e. the edges of the optic nerve sheath. Thus, the image may be segmented to separately identify the eye, the optic nerve sheath, and background areas, thereby enabling an eye circle and the edges of the optic nerve sheath to be identified.

The principal direction may be a direction aligned with a centre-line of the boundary features identified by the use of computer implemented image processing techniques, and thus a direction intended to align with the centre-line of the optic nerve sheath (or optic nerve). The computer implemented image processing techniques may identify at least two boundaries extending along the sides of the optic nerve sheath by reference to changes visible in the ultrasound images (e.g. changes of intensity). It will be appreciated that the computer implemented image processing techniques are of course operating on the underlying image data, but it is convenient to describe this in terms of identification of characteristics that are visible when this image data is displayed as an image. Typically the outer edges of the optic nerve sheath can be seen as changes in the image, and hence these can form the boundary features. The identification of the boundaries may include identifying points along the extent of two sides of the optic nerve sheath and fitting two vectors to these points to define directions extending along the two sides of the optic nerve sheath. The method may then include determining an average of the two vectors in order to determine the principal direction, which will then align with the centre of the boundary features in the images. It will be appreciated that these steps involve the use of image data for image planes that are aligned with the principal direction.

When the principal direction aligns with the centre of the optic nerve then a cross-section of the optic nerve sheath (or the optic nerve) can be determined by identifying in a suitable ultrasound image two opposite positions on the relevant boundary features of the ON or ONS, the positions being perpendicular to the same location on the axis represented by the principal direction.

The method may include calculation of the diameter of the optic nerve sheath. In some examples, this is done automatically by computer implemented image processing techniques using images obtained after the alignment with the principal direction. It will be appreciated that when images are correctly aligned with a principal direction of the optic nerve sheath then a horizontal imaging plane will accurately cut through a diameter. In some examples, the method includes diameter measurements at different distances along the principal direction in order to find a maximum diameter.

A set of steps for finding a maximum diameter may be carried out as follows:

(i) Collecting a set of coordinates representative of the cross-section of the optic nerve sheath boundary, and fitting a circle model to these points.

(ii) Measuring diameter as the distance from one side of the boundary to the other along the normal of the principal direction, which is typically done in an area 2 mm to 10 mm below the eye circle, optionally 2 mm to 5 mm.

(iii) Recording diameter at the first measurement, and in subsequent measurements, replacing the recorded maximum diameter if the subsequently measured diameter is larger.

Optionally, if the subsequently measured diameter is smaller than the recorded maximum diameter then the difference between the current diameter and the maximum diameter may be used to determine if the ultrasound image plane is cutting through the true centre of the nerve. This can be done since the observed maximum diameter is assumed to be the true diameter of the optic nerve sheath, and therefore is a target diameter. This optional step requires the probe to perform a sweep over the eye in order to observe the maximum optic nerve sheath diameter. The images of the transducer can be reconstructed to form a three dimensional image volume, by image processing techniques (e.g image stitching methods) or by the aid of a positioning device.

The method may include machine learning techniques in relation to algorithms that are used for automated processing of the images to find the boundaries of the optic nerve sheath. Thus, an algorithm for identification of the boundary feature may be developed via machine learning techniques, such as by learning from sample images with the boundary features identified to the machine learning process by a skilled human operator. The method may alternatively or additionally use machine learning techniques for the automated processing of images obtained after alignment, such as for diameter measurement, for monitoring pulsatile dynamics of the optic nerve sheath as discussed further below, or for automated determination of healthy patients verses unhealthy patients, for example in relation to specific conditions such as papilledema.

Thus, in one application of the proposed alignment technique, images of the optic nerve sheath obtained via this alignment process are used in a method for non-invasively calculating a marker indicating possibly increased intracranial pressure of a patient.

In one example, a method for non-invasively calculating a marker indicating possibly increased intracranial pressure of a patient may comprise obtaining images of the optic nerve sheath after alignment with the principal direction, and then determining a measure of stiffness of the optic nerve sheath. In some examples, the method includes quantifying "pulsatile dynamics" of the optic nerve sheath, i.e. factors relating to dynamic movements arising from pulsatile motions of the body, such as by monitoring dynamic properties of the optic nerve sheath, and/or of nearby regions, based on motion of the optic nerve sheath over a period of time. The measured dynamic properties may then be used to determine a measure of stiffness of the optic nerve sheath. A measure of stiffness, such as one determined via the quantified pulsatile dynamics, can be used to obtain the marker indicating possibly increased intracranial pressure by associating increased stiffness with increased intracranial pressure.

In alternative or additional steps, the method for non-invasively calculating a marker indicating possibly increased intracranial pressure of a patient may comprise detecting cardiac pulse related motion of the optic nerve sheath by monitoring displacements on each side of the optic nerve sheath; using a difference between the displacements on each side of the optic nerve sheath to determine a measure of stiffness of the optic nerve sheath; and using the detected displacements and the measure of stiffness to obtain the marker indicating possibly increased intracranial pressure by associating increased stiffness with increased intracranial pressure. The monitoring of dynamic properties may be carried out during motion of the optic nerve sheath over a period of time including one or more cardiac cycles. Optionally, the method may utilise motions of the optic nerve sheath that are caused by cardiac and/or respiratory motion in order to obtain the required dynamic properties. Thus, the motion of the optic nerve sheath caused by cyclic body functions such as cardiac and/or respiratory motion, such as a pulsing variation in diameter, form, or position of the optic nerve sheath, may be used to derive a measure of stiffness that provides an indicator of intracranial pressure. The motions of the optic nerve sheath may be investigated with reference to variations occurring during a single cardiac cycle. Alternatively or additionally the method may include inducing a displacement of the optic nerve sheath and/or an associated biological response of the patient in order to prompt motion of the optic nerve sheath.

When being used to carry out measurements of dynamic properties, the method may include evaluating whether the operator has acquired an image sequence of sufficient quality, i.e. determining if a suitably aligned position is held over a particular time period, which can be a time period deemed sufficiently long to allow processing of the dynamic properties. This may be a time period covering a certain number of cycles for a cyclic body function such as those discussed above.

The monitoring of dynamic properties may comprise detecting variation in time for displacements of points on the optic nerve sheath as they vary with time at two locations around the optic nerve sheath or in the region surrounding the optic nerve sheath. For example, the method may use at least two locations on the boundary feature identified during the alignment process. Multiple sets of pairs of locations may be used. The method may make use of average displacements from several locations at the one side of the optic nerve sheath, and average displacements from several locations at the one side of the optic nerve sheath. The measure of stiffness of the optic nerve sheath may then include obtaining a parameter of deformability (Δ) based on the displacements. The parameter of deformability (Δ) is calculated according to the equation:

$$\Delta = \frac{|d_A - d_B|}{d_A + d_B}$$

wherein $d_A$ and $d_B$ represent the displacements at the two locations.

The method may comprise performing a Fourier analysis of the motion pattern of the optic nerve sheath (such as via points on the boundary feature) in a direction perpendicular to a longitudinal axis of the optic nerve sheath.

The method may include obtaining images of the eye circle after alignment with the principal direction, and automated processing of the images in order to determine if the patient's eye exhibits symptoms related to a particular condition of the eye. As mentioned above, one condition that may be the subject of this type of image processing is papilledema. The method may hence comprise automated quantification of papilledema using the images of the eye circle obtained after alignment with the principal direction. This may include processing of the images to identify the shape of the eye and to then determine a measurement of papilledema with reference to the shape and size of formations at the back of the eye.

As is known, papilledema is optic disc swelling that may be caused by increased intracranial pressure due to any cause. Thus, as with the method above it provides another marker for intracranial pressure. Different markers for intracranial pressure may advantageously be used together, with some form of combined assessment allowing for a fail-safe arrangement (such as an "or" combination of two markers) or a more accurate assessment of the possible risk of intracranial pressure, especially in relation to patients that might be borderline for one marker.

The automated quantification of papilledema using computer implemented processing of ultrasound images is considered novel and inventive in its own right and therefore, viewed from a second aspect, the invention provides: a computer implemented method for automated quantification of papilledema comprising: obtaining ultrasound images of a region of interest including the eye circle and optic nerve; using computer implemented image processing techniques, identifying a principal direction of the optic nerve and the curve of the eye circle; based on the intersection of the principal direction and the curve of the eye circle, identifying an area of the eye circle relevant to papilledema; and, using an image processing algorithm, finding one or more parameter(s) relating to the shape and/or size of a formation at the back of the eye in the area of the eye circle known to exhibit papilledema, and thereby quantifying the papilledema.

As will be appreciated, this method may stand alone, or it may be combined with the method for alignment of the image plane of the ultrasound probe set out above, including optional features thereof. It is preferred for the method of the second aspect to be fully automated in terms of the processing of the ultrasound images. Thus, after the point of obtaining the images the method may be performed by computer without any human interaction. This can allow for a quantification of papilledema to be provided to the user without the need for any skill or training in relation to the interpretation of ultrasound images. Moreover, if combined with a suitable apparatus and/or method for easily obtaining ultrasound images with the required alignment, such as the method of the first aspect, then the entire process of obtaining the ultrasound images and determining the one or more parameter(s) enabling quantification of the papilledema can be carried out without any in-depth training or experience.

In relation to the step of identifying a principal direction of the optic nerve and the curve of the eye circle, the method may include steps as detailed above for the first aspect or optional features thereof, with the principal direction hence being as above.

Alternatively the method may include (e.g. for images obtained without the use of the above alignment process): identifying a boundary of the optic nerve sheath, calculating the horizontal position and orientation of the optic nerve, calculating co-ordinates along the two sides of the optic nerve sheath, fitting a line to the co-ordinates to find the direction along each of the two sides, for example by calculating a principal eigenvector for each side and finding an average of the two directions to thereby find the principal direction of the optic nerve sheath. This might be done by, for example, determining a midpoint vector between two vectors and defining the direction as being the direction in which the midpoint vector extends. This process may be performed using a neural network, for example a deep convolutional neural network.

Another possibility is to use one or both of the two lines representing the boundary of the optic nerve as a principal direction, determining the intersection of an extension of the line(s) with the eye circle, and identifying the area of the eye circle relevant to papilledema as being the area adjacent to a line, or between the two lines where both lines are used. That is, the papilledema can be found within the area in between the points where the two lines from the boundary features intersect with the eye circle. The step of identifying the curve of the eye circle may include the use of image processing to determine relevant features of the ultrasound images, such as via segmentation of one or more of the ultrasound image(s) using a neural network, for example a deep convolutional neural network. This can be the same neural network used for determining the principal direction of the optic nerve. The method may include calculating the position of the eye circle, for example by the use of machine learning techniques, such as via an algorithm devised by machine learning with reference to a set of ultrasound images with the eye circle position having been identified by a suitably skilled person. Point coordinates of the bottom of the eye may be calculated based on the segmentation, with a circle or partial circle being fitted to the points, for example by using the Kasa circle fitting algorithm.

The step of identifying an area of the eye circle relevant to papilledema is based on the intersection of the principal direction and the curve of the eye circle, and may include identifying an area centred on the point of intersection. For example, the method may comprise identifying the area as being within a certain distance of the intersection of the principal direction and the eye circle, with the distance being measured circumferentially along the extent of the eye circle. In one example the certain distance is in the range 1 mm to 5 mm to either side of the intersection point, for example it may be 3 mm to either side of the intersection point.

Based on the identified area, the method includes using an image processing algorithm to find one or more parameter(s) relating to the shape and/or size of a formation at the back of the eye in the area of the eye circle known to exhibit papilledema. As is known, the papilledema presents itself as a "bump" at the back of the retina extending forward from the back of the eye into the eye, with a bump in this region hence being seen as a possible papilledema. The parameter(s) may include one or more of: the maximum extent that such a possible papilledema protrudes from the eye circle; an area of the possible papilledema; a volume of the possible papilledema; and/or an extent of the possible papilledema along the circumference of the eye circle. The parameter(s) may be measured in absolute terms, such as via a mm value, or they may be measured in relative terms, such as in relation to a reference measure, for example the diameter of the patient's eye circle or the diameter of the optic nerve or optic nerve sheath. The parameter(s) can be used directly to quantify the papilledema, and/or a combination of parameters may be made, and/or the parameters may be compared to a threshold value to determine if the patient should be deemed to be at risk or healthy in relation to papilledema.

In the case where the parameter (or one of the parameters) includes the maximum extent that a possible papilledema protrudes from the eye circle then the method may include, for multiple points along the eye circle in the identified area, determining an inner edge of the possible papilledema and finding the distance between the back of the eye circle and the inner edge of the possible papilledema. The multiple points may include all points along the eye circle, or a selection of points spaced apart from one another. This may be done by use of any suitable edge detection method, such as via a step edge model. The method may include sampling image intensity along a line starting from the outer edge of the eye circle and extending inwardly in the normal direction of the eye circle. In one example, the line extending inwardly from the outer edge of the eye circle may have a length in the range 1 mm to 4 mm, for example it may be about 2 mm in length. It will be understood that by finding the edge of the possible papilledema along all such lines for the identified area then it is possible to capture a set of coordinates that define the shape and size of the possible papilledema. The method may include determining the maximum extent of the possible papilledema along any one of these lines and using that maximum extent as the parameter enabling quantification of the papilledema.

Thus, in a particular example the method may include finding the multiple points along the eye circle within 3 mm either side of the intersection between the principal direction and the eye circle, and then sampling image intensities along a line of about 2 mm in length extending inwardly in the normal direction of the eye circle.

Viewed from a third aspect, the present invention provides a computer programme product comprising instructions that, when executed, will configure a computer system including an ultrasound imaging device to carry out the method of the first aspect.

Thus, the computer programme product of this aspect may configure the computer system to use an ultrasound probe of the ultrasound imaging device to obtain images of anatomical structures in the region of the eye and across a region of interest expected to include the optic nerve; process the images of said region of interest via computer implemented image processing techniques in order to identify boundary features in the images that are representative of the boundaries of at least one of the optic nerve and the optic nerve sheath; use the identified boundary features of the imaged structure to determine a principal direction extending along the length of the optic nerve and the optic nerve sheath; identify at least first and second points on the identified boundary features, wherein the first and second points are at different locations along the extent of the principal direction; and guide the user to move the probe or control the probe to move the image plane of the probe in order to rotate the image plane of the probe until the first and second points are aligned, and thereby guide the image plane of the probe to a required orientation for alignment of the ultrasound image with the principal direction.

The instructions may configure the computer system to carry out further features of the method as set out above in relation to the first aspect and optionally also the second aspect.

Viewed from a fourth aspect, the present invention provides a computer programme product comprising instructions that, when executed, will configure a computer system including an ultrasound imaging device to carry out the method of the second aspect.

Thus, the computer programme product of this aspect may configure the computer system to use an ultrasound probe of the ultrasound imaging device to obtain images of a region including the eye circle and optic nerve; using computer implemented image processing techniques, identify a principal direction of the optic nerve and the curve of the eye circle; based on the intersection of the principal direction and the curve of the eye circle, identify an area of the eye circle relevant to papilledema; and, using an image processing algorithm, find one or more parameter(s) relating to the shape and/or size of a formation at the back of the eye in the area of the eye circle known to exhibit papilledema, and thereby quantifying the papilledema.

The instructions may configure the computer system to carry out further features of the method as set out above. The instructions may configure the computer system to carry out the methods of both the first aspect and the second aspect, and thus the computer programme products of the third aspect and fourth aspect may be combined.

Viewed from a fifth aspect, the invention provides an apparatus for alignment of an ultrasound image obtained with an ultrasound probe for obtaining images in relation to the eye, the apparatus comprising:

> an ultrasound imaging device including the ultrasound probe; and
> a computer system configured to carry out the method of the first aspect, the computer system optionally having been so configured by the computer programme product of the third aspect.

Thus, the computer system of this aspect may be configured to use the ultrasound probe of the ultrasound imaging device to obtain images of anatomical structures in the region of the eye and across a region of interest expected to include the optic nerve; process the images of said region of interest via computer implemented image processing techniques in order to identify boundary features in the images that are representative of the boundaries of at least one of the optic nerve and the optic nerve sheath; use the identified boundary features of the imaged structure to determine a principal direction extending along the length of the optic nerve and the optic nerve sheath; identify at least first and second points on the identified boundary features, wherein the first and second points are at different locations along the principal direction; and guide the user to move the probe or control the probe to move the image plane of the probe in order to rotate the image plane of the probe until the first and second points are aligned, and thereby guide the image plane of the probe to a required orientation for alignment of the ultrasound image with the principal direction.

The computer system may optionally be configured to carry out further features of the method as set out above.

Viewed from a sixth aspect, the invention provides an apparatus for automated quantification of papilledema, the apparatus comprising:

> an ultrasound imaging device including an ultrasound probe; and
> a computer system configured to carry out the method of the second aspect, the computer system optionally having been so configured by the computer programme product of the fourth aspect.

Thus, the computer system of this aspect may be configured to use the ultrasound probe of the ultrasound imaging device to obtain images of a region including the eye circle and optic nerve; using computer implemented image processing techniques, identify a principal direction of the optic nerve and the curve of the eye circle; based on the intersection of the principal direction and the curve of the eye circle, identify an area of the eye circle relevant to papilledema; and, using an image processing algorithm, find one or more parameter(s) relating to the shape and/or size of a formation at the back of the eye in the area of the eye circle known to exhibit papilledema, and thereby quantifying the papilledema.

The computer system may optionally be configured to carry out further features of the method as set out above. The computer system may be configured to carry out the methods of both the first aspect and the second aspect, and thus the apparatus of the fifth aspect and the apparatus of the sixth aspect may be combined, with the computer system having the capabilities of both aspects.

Certain embodiments will now be described by way of example only and with reference to the accompanying drawings, in which:

> Figure 1 illustrates a schematic of an ultrasound image that is segmented into background, eye and optic nerve sheath;
> Figure 2 shows an example of coordinates along the side of the optic nerve sheath;
> Figure 3 shows the addition of principal eigenvectors of each side of the optic nerve sheath, along with a principal direction calculated as the average of the two eigenvectors;
> Figure 4 shows points along the eye circle and a circle fitted to those points;
> Figure 5 is an example screenshot of a graphical user interface for guiding manual alignment of an ultrasound probe;
> Figure 6 shows the graphical user interface with alignment of the probe with the principal direction;
> Figure 7 is an ultrasound image of an eye including papilledema with markings overlaid showing steps used in quantification of the papilledema
> Figure 8 is a schematic diagram of a segmented ultrasound image similar to that of Figures 1 to 4, with lines added indicating a proposed method for quantification of the papilledema similar to Figure 7.

The method and apparatus described herein may be used to guide a user to acquire an optimal view of an optic nerve sheath (ONS) by correctly aligning an ultrasound image obtained with an ultrasound probe with a principal direction of the ONS. This may be desired for example so that the intracranial pressure (ICP) of a patient can be measured non-invasively using the ultrasound image. Preferred aspects of an optimal image may include that the ONS is located in the horizontal centre of the image, and/or the ONS centreline (or mid-axis) is aligned with the vertical axis of the image, and/or the image plane cuts through the centre of the ONS.

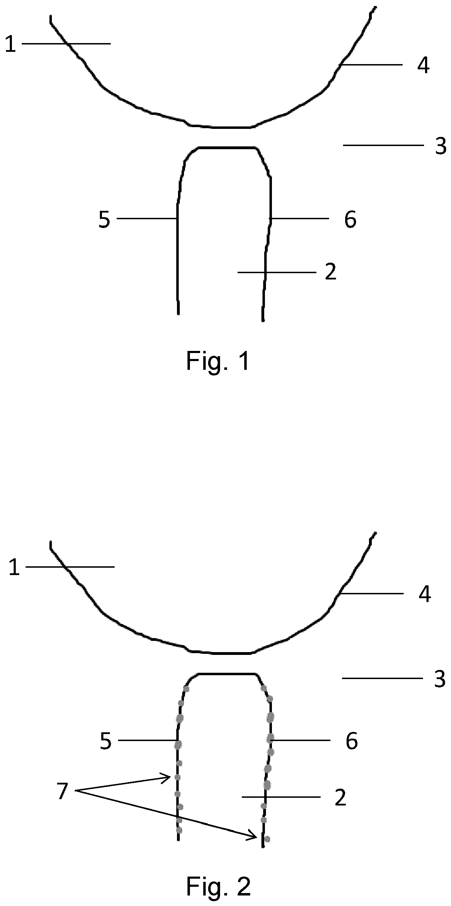

The proposed method proceeds as follows. An ultrasound probe is placed in a suitable location for obtaining images of anatomical structures in the region of the eye and across a volume/Region of Interest (ROI) expected to include the optic nerve and/or the optic nerve sheath. Images of the volume/ROI are obtained using the ultrasound probe. For every incoming image frame from the ultrasound probe the following processing steps are performed, as illustrated in Figures 1 to 3. These processing steps are preferably performed by computer implemented image processing techniques.

Figure 1 illustrates the first step of the proposed method wherein the image is segmented into three classes: eye 1, optic nerve and optic nerve sheath (ONS) 2, and background 3. It will be understood that more or fewer classes may be required depending on the structures present in the image. The segmentation may be performed using a neural network, for example using a deep convolutional neural network. Segmentation of the image is beneficial as it enables boundary features 4, 5, 6 in the image to be identified. In the schematic of Figure 1, boundary features in the image that are representative of the ONS 2 and the eye 1 are identified. In general, it is preferred to identify boundary features representative of at least one of the ONS 2 and the optic nerve. Machine learning techniques may be used in relation to algorithms for automated processing of the images to find the boundary features. In an example the boundary between the eye 1 and the background 3 is represented by an eye circle 4, i.e. a circular arc around the back of the eye 1 when the ball of the eye 1 is seen in two dimensions, and the boundary between the ONS 2 and the background 3 is represented by first and second sides 5, 6.

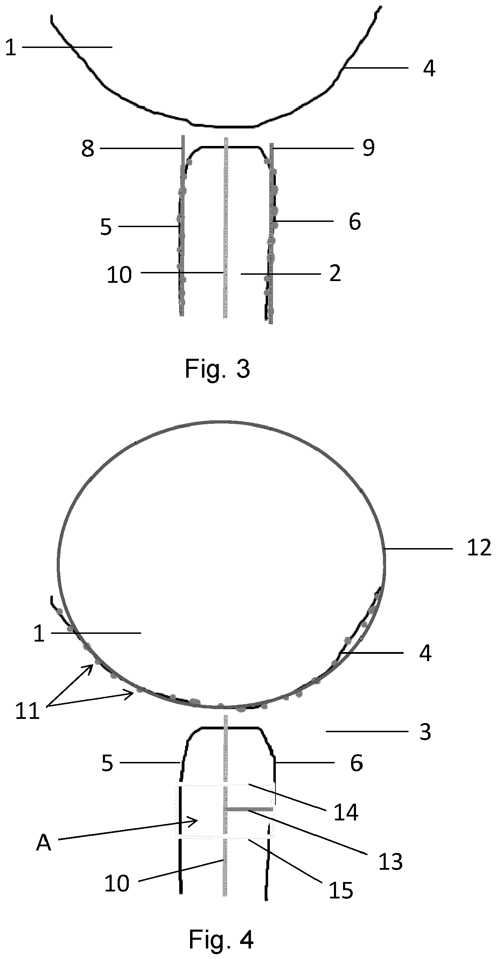

The boundary features 4, 5, 6 are then used to determine a principal direction 10 extending along the length of the optic nerve and/or the ONS 2, as shown in Figures 2 and 3. A first set of points 7 along the extent of the first and second sides 5, 6 are identified and coordinates of the first set of points 7 are calculated. The first set of points 7 may be identified by reference to changes in the ultrasound image, for example changes of intensity. A first principal eigenvector 8 is fitted to the points in the first set of points 7 which lie along the first side 5, and similarly a second principal eigenvector 9 is fitted to the points in the first set of points 7 which lie along the second side 6, as illustrated in Figure 3. The first and second principal eigenvectors 8, 9 define directions extending along the first and second sides 5, 6 of the ONS 2 respectively. In order to calculate the principal eigenvectors 8, 9, at least two points on each of the first side 5 and the second side 6 are identified. A centre-line 10 of the ONS 2 is calculated from an average of the first and second principal eigenvectors 8, 9. The centre-line 10 defines the principal direction 10 of the ONS 2 and extends along the length of the ONS 2 and/or the optic nerve. Thus, the principal direction 10 with which the ultrasound image is desired to be aligned is identified.

The principal direction 10 may be used to determine whether the ONS 2 is located in the horizontal centre of the ultrasound image and/or whether the ONS 2 is vertically aligned with the image, in accordance with preferred aspects of an optimal image as described above.

The diameter of the ONS 2 may then be measured by the following steps as illustrated in Figure 4. It may be desired to find the diameter in order to determine if the ultrasound image plane is cutting through the true centre of the ONS 2, and/or to perform processing techniques to determine conditions of the eye such as stiffness of the ONS 2 as explained previously. It is preferred that the following steps are carried out automatically, e. g. by computer implemented image processing techniques.

A second set of points 11 along the eye circle 4 are identified and coordinates of the second set of points 11 are calculated. A fitted circle 12 is fitted to the second set of points 11 using any suitable fitting method, such as via a least squares method, for example using the Kasa circle fitting algorithm. The fitted circle 12 defines the curve of the eye circle 4.

A normal 13 extends from the centre-line 10 in a perpendicular direction to the second side 6, although the normal could alternatively extend to the first side 5. The normal 13 is positioned within an area A, wherein the boundaries of area A may be defined by the first and second principal eigenvectors 8, 9, a top side 14, and a bottom side 15. The top side 14 may be positioned at a first predetermined depth below the fitted circle 12, and the bottom side 15 may be positioned at a second predetermined depth below the fitted circle 12. The first predetermined depth may be smaller than the second predetermined depth, for example the first predetermined depth may be 3mm and the second predetermined depth may be 6mm. The top side 14 and the bottom side 15 may extend in a direction perpendicular to the centre-line 10, i.e. parallel to the normal 13.

The diameter of the ONS 2 is then measured as the distance from the first side 5 to the second side 6 along the normal 13 of the centre-line 10, within the area A.

An observed maximum diameter of the ONS 2 is assumed to be the true diameter of the ONS 2. Therefore it may be desired to find the maximum diameter in order to ensure that the ultrasound image plane is cutting through the true centre of the ONS 2, in accordance with one of the features of an optimal image as described above. This may require an initial sweep of the ultrasound probe over the eye in order to observe the maximum diameter. The sweep may involve taking a plurality of ultrasound images through different image planes and measuring the diameter of the ONS 2 in each different image plane. The diameter may also be measured at different distances along the principal direction 10 in order to find the maximum diameter of the ONS 2. During the sweep if a measured diameter in a current image plane is larger than a previously determined maximum diameter, the maximum diameter may be replaced with the value of the current diameter.

Once the maximum diameter has been determined, the current diameter can be compared with the maximum diameter to ascertain whether the image plane is cutting through the true centre of the ONS 2.

It has now been ascertained whether the image plane of the ultrasound probe is aligned correctly such that the principal direction 10 of the ONS 2 is vertically and horizontally aligned with the image, and such that the image plane cuts through the true centre of the ONS 2. If the image plane of the ultrasound probe is not aligned correctly, it may be desired to adjust the alignment. In one example of the proposed method a graphical representation of the position of the ONS 2 in relation to the ultrasound image plane is used to guide a user to manually adjust the image plane of the probe by rotating the probe. The method may include identifying at least two points on the ONS boundary features 5, 6, such as two cross-sections of the ONS 2 and moving the probe to align those points thereby aligning the probe with the principal direction 10, as described in more detail below.

In the illustrated example, first and second cross-sections of the identified boundary features are identified, wherein the first cross-section is located on the ONS 2 at a first predetermined depth below the eye circle 4 and the second cross-section is located on the ONS 2 at a second predetermined depth below the eye circle 4. For example the first predetermined depth may be 3mm and the second predetermined depth may be 6mm. In order to determine the relative alignment of the first and second cross-sections a graphical user interface or visual display can be used, which allows the user to receive feedback in relation to the degree of alignment of the cross-sections. The graphical user interface may also help guide the user to rotate the ultrasound probe into an aligned position such that an optimal image may be acquired.

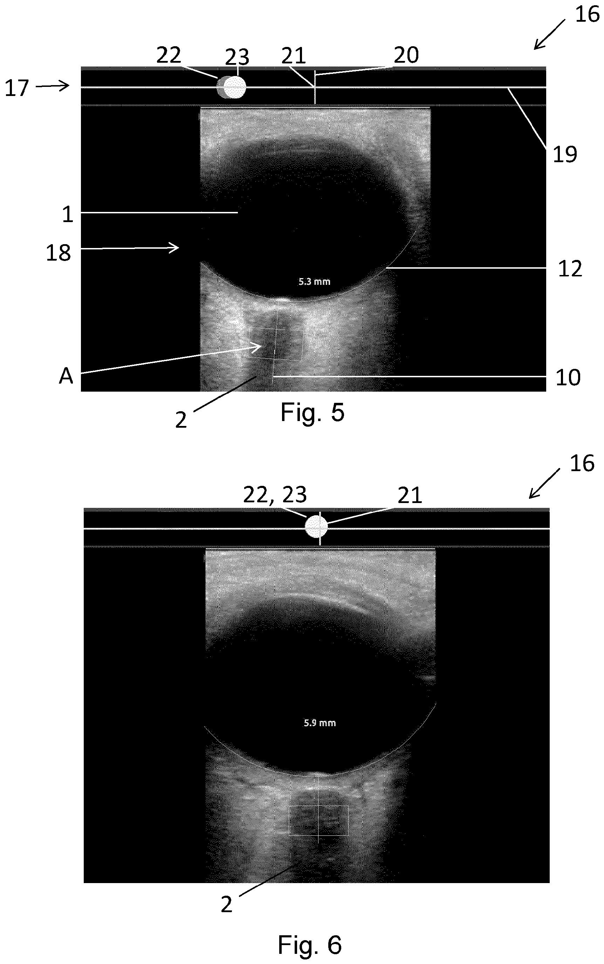

Figures 5 and 6 show an example graphical user interface 16 which may guide the user in this way. The interface 16 includes a guidance bar 17 and an ultrasound image 18. In the ultrasound image 18, the eye 1 and the ONS 2 are visible. The image 18 is overlaid with markings showing some of the boundary features identified by the steps illustrated in Figures 1 to 4. These markings may include the fitted circle 12, the centre-line 10, and the area A. The guidance bar 17 illustrates a projection of the ultrasound image plane. The guidance bar 17 may include guidance markings, for example a horizontal guidance line 19 and a vertical guidance line 20 which intersect at an intersection 21. The horizontal guidance line 19 represents the ultrasound image plane, and the vertical guidance line 20 is aligned with the centre of the ultrasound image. The guidance bar 17 may further include guidance markings, for example circles, which represent the identified cross-sections of the ONS 2. The markings may represent projections of the cross-sections on the ultrasound image plane. For example, a first circle 22 may represent a first cross-section along the principal direction 10 at a depth of 3mm below the eye, such that the position of the first circle 22 along the horizontal guidance line 19 corresponds to the horizontal position of the first cross-section of the ONS 2 in the image. Similarly, a second circle 23 may represent a second cross-section along the principal direction 10 at a depth of 6mm below the eye, such that the distance of the second circle 23 along the horizontal guidance line 19 corresponds to the horizontal position of the second cross-section. Although the Figures illustrate guidance lines and circular markings, alternative types of markings may be used to represent the relevant features.

The first and second circles 22, 23 can be used to help the user visualise the position and orientation of the ONS 2 within the image plane. For example when the first and second circles 22, 23 are not overlapping, the ONS 2 is not vertically aligned in the image. When the circles 22, 23 are not centred on the vertical line 20, the ONS 2 is not horizontally centred in the image. Figure 5 shows an example graphical user interface in this case, i.e. when the ONS 2 is not horizontally centred and not vertically aligned. As shown in the ultrasound image 18, the centre-line 10 of the ONS 2 is displaced to the left of the horizontal centre of the ultrasound image and is slanted at an angle to the vertical direction.

Once the user has determined the relative position and/or orientation of the cross-sections, it may be desired to move the probe to correctly align the cross-sections. The probe is thus rotated and/or moved in translation until the cross-sections are aligned, i.e. until the first and second circles 22, 23 overlap completely or substantially, thereby determining a required orientation of the ultrasound probe for alignment with the principal direction. The rotating of the probe may occur manually as discussed above and/or may be done with an actuation mechanism. The alignment of the image relative to the defined cross sections can also be done by electronic steering of the beams of the transducer array, wherein the activations of the elements of the transducer array or the timing of any signal fed to the transducer elements is electronically controlled. The alignment of the image may also involve rotating the image plane of the probe whilst the physical structure of the probe remains in one place.

The first and second circles 22, 23 may also be used to ensure the principal direction is centrally aligned with the ultrasound image by using the vertical guidance line 20 on the guidance bar 17. The image plane of the ultrasound probe is moved until the first and second circles 22, 23 representing the two cross-sections are centred on the vertical guidance line 20, at which point the ONS 2 is centrally positioned within the image frame.

Figure 6 shows a graphical user interface when the ultrasound probe is correctly aligned with the principal direction 10 and the ONS 2 is horizontally centred. As shown on the guidance bar 17, the first and second circles 22, 23 are overlapping substantially and are positioned on the intersection 21. Correspondingly, as shown in ultrasound image 18, the ONS 2 is vertically aligned and horizontally centred in the image. This demonstrates an optimal view of the ONS 2.

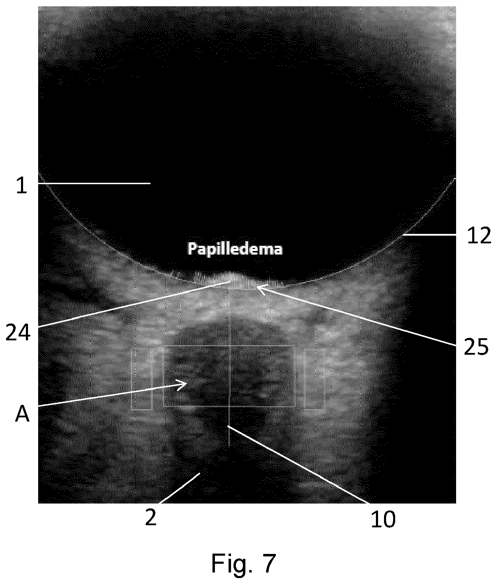

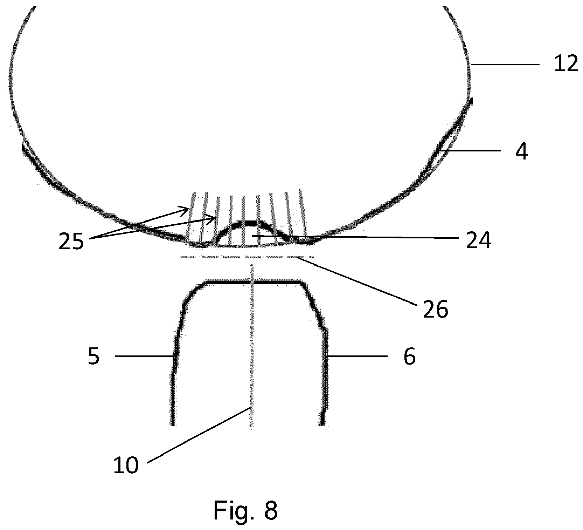

The method may further include obtaining images of the eye and/or the ONS 2 after alignment of the image plane of the probe with the principal direction 10. The images may be used for example for automated assessment of the diameter of the ONS 2 as described above, and/or for determining a measure of stiffness of the ONS 2 as a marker for increased intracranial pressure. The method may comprise automated processing of the images in order to determine if the patient's eye exhibits symptoms related to a particular condition of the eye or the optic nerve complex, in particular identification and/or quantification of papilledema. A proposed method for quantification of papilledema is described in more detail below with reference to Figures 7 and 8.

As previously discussed, one known way that papilledema presents itself is as a formation, which is typically in the form of a "bump" or protrusion, located at the back of the eye extending forward into the eye. The formation may be visible on an ultrasound image of an eye, in particular in the region of the eye circle 4.

According to the proposed method, the area of the eye circle 4 relevant to papilledema is identified based on the intersection between the principal direction 10 and the curve of the eye circle, wherein the curve is defined by the fitted circle 12 as previously described. Figure 7 shows an ultrasound image of an eye 1 having a formation 24 which is characteristic of papilledema. Once papilledema is identified, it may be desired to perform quantification. One technique for quantifying papilledema is to find the maximum extent of the formation 24 into the eye. Other possibilities include determining the area or volume of the formation 24. By way of example, further details are given below with reference to finding the maximum extent of the formation 24. The maximum extent of the formation 24 may be defined as the maximum distance between the back of the eye, i.e. the fitted circle 12, and an inner edge of the formation 24.

The ultrasound image in Figure 7 is overlaid with markings which include the eye circle 4, centre-line 10 and area A as identified by the steps described previously. The markings further include a series of lines 25 that extend from the fitted circle 12 to an inner edge of the formation 24 in a direction radially inward toward the centre of the eye 1. The inner edge of the formation 24 is the boundary between the formation 24 and the eye 1. The lines 25 may have a predetermined length, for example 2mm. The lines 25 may be drawn from every point that is along the fitted circle 12 within a pre-determined distance (shown by a dashed line 26 in Figure 8) either side of the intersection between the principal direction 10 and the fitted circle 12. The pre-determined distance may be 3mm for example.

Along every line 25, image intensities are sampled and a change in image intensity is used to determine a coordinate of the inner edge of the formation 24. The inner edge coordinate may be estimated using an existing edge detection method such as the step edge model. Therefore by finding the inner edge coordinate for all lines 25 on the fitted circle 12, a set of coordinates that defines the shape and size of the formation 24 is collected. The maximum extent of the formation 24 along any of lines 25 is determined, and said maximum extent is used as the parameter enabling identification and/or quantification of the papilledema.

The parameter can be used directly to quantify the papilledema, and/or in combination with other parameters, and/or the parameters may be compared to a threshold value to determine if the patient is deemed to be at-risk or healthy in relation to papilledema.

As described previously, there are other further methods that may be carried out using ultrasound images after alignment of the ultrasound image with the principal direction 10. In one example, a measure of stiffness of the optic nerve sheath can be determined as a marker for increased intracranial pressure, for example by monitoring dynamic properties of the optic nerve sheath, and/or of nearby regions, based on motion of the optic nerve sheath over a period of time. WO 2016/193168 describes an example of a method that may be used in relation to images obtained via the proposed alignment method. When being used to carry out measurements of dynamic properties, the method may include evaluating whether the operator has acquired an image sequence of sufficient quality, i.e. that the correct view of the ONS 2 is held over a period necessary to process the dynamic properties.

The following clauses set out features of the present disclosure which may not presently be claimed but which may form basis for future amendments and/or a divisional application.

    1. A method for alignment of an ultrasound image obtained by an ultrasound probe for obtaining images in relation to the eye, the method comprising:

        placing the ultrasound probe in a suitable location for obtaining images of anatomical structures in the region of the eye and across a region of interest expected to include the optic nerve;
        obtaining images of said region of interest using the ultrasound probe;

processing the images of said region of interest via computer implemented image processing techniques in order to identify boundary features in the images that are representative of the boundaries of at least one of the optic nerve and the optic nerve sheath;

using the identified boundary features of the imaged structure to determine a principal direction extending along the length of the optic nerve and the optic nerve sheath;

identifying at least first and second points on the identified boundary features, wherein the first and second points are at different locations along the principal direction; and

rotating the image plane of the probe until the first and second points are aligned in the image, and thereby determining a required orientation of the image plane of the probe for alignment of the ultrasound image with the principal direction.

2. A method as recited in clause 1, wherein the probe includes an actuation mechanism for rotating the probe and the method comprises using the actuation mechanism to rotate the probe to align the first and second points; and/or wherein electronically controlled activations of the transducer array elements are used to steer the image plane to align the first and second points.

3. A method as recited in clause 1, wherein the probe is arranged to be handled manually and the step of rotating the probe is performed by the user manually rotating the probe to align the first and second points.

4. A method as recited in clause 1, 2 or 3, comprising obtaining images of the optic nerve sheath after alignment of the ultrasound image with the principal direction, and using the images for at least one of: automated assessment of the diameter of the optic nerve sheath; and determining a measure of stiffness of the optic nerve sheath as a marker for increased intracranial pressure.

5. A method as recited in any preceding clause, comprising obtaining images of the optic nerve sheath and the eye circle after alignment of the ultrasound image with the principal direction, and using the images for quantification of papilledema.

6. A method as recited in any preceding clause, wherein the two points are two cross-sections of the identified boundary features and the method comprises alignment of the centre of the ultrasound image with the principal direction by ensuring that the two cross-sections are aligned with each other via rotation of the image plane of the probe whilst also ensuring that they are centrally placed within the ultrasound image from the probe by translating the probe.

7. A method as recited in any preceding clause, wherein the two points are two cross-sections of the identified boundary features and the method comprises providing feedback to the user in relation to the degree of alignment of the cross-sections using a visual display with representations of the two cross-sections.

8. A method as recited in clause 7, wherein the visual display uses two markings, such as circles, to represent the two cross-sections and a further marking, such as a line, to represent the centre of the ultrasound image.

9. A method as recited in any preceding clause, wherein the two points on the identified boundary features are spaced apart by at least 2 mm along the principal direction.

10. A method as recited in any preceding clause, wherein the two points on the identified boundary features are at distances below the eye circle of about 3mm and 6 mm.

11. A method as recited in any preceding clause, wherein the principal direction is a direction aligned with a centre-line of the boundary features identified by the use of computer implemented image processing techniques, wherein the computer implemented image processing techniques identify the directions of two boundaries extending along the sides of the optic nerve sheath by identifying points along the two sides of the optic nerve sheath and fitting two vectors to these points to define directions extending along the two sides of the optic nerve sheath; and wherein the principal direction is an average of the two vectors in order to determine the principal direction.

12. A method as recited in any preceding clause, comprising determining diameter measurements for the optic nerve sheath at different distances along the principal direction in order to find a maximum diameter of the optic nerve sheath.

13. A method as recited in any preceding clause, comprising the use of machine learning techniques in relation to algorithms for automated processing of the images to find the boundaries of the optic nerve sheath.

14. A method for non-invasively calculating a marker indicating possibly increased intracranial pressure of a patient, the method comprising obtaining images of the optic nerve sheath after alignment of an ultrasound image obtained with an ultrasound probe with the principal direction in accordance with the method of any preceding clause; quantifying pulsatile dynamics of the optic nerve sheath by monitoring dynamic properties of the optic nerve sheath, and/or of nearby regions, based on motion of the optic nerve sheath over a period of time; using the measured dynamic properties to determine a measure of stiffness of the optic nerve sheath; and using the quantified pulsatile dynamics, including the measure of stiffness, to obtain the marker indicating possibly increased intracranial pressure by associating increased stiffness with increased intracranial pressure.

15. A method for non-invasively calculating a marker indicating possibly increased intracranial pressure of a patient, the method comprising obtaining images of the optic nerve sheath after alignment of an ultrasound image obtained with an ultrasound probe with the principal direction in accordance with the method of any of clauses 1 to 13; detecting cardiac pulse related motion of the optic nerve sheath by monitoring displacements on each side of the optic nerve sheath; using a difference between the displacements on each side of the optic nerve sheath to determine a measure of stiffness of the optic nerve sheath; and using the detected displacements and the measure of stiffness to obtain the marker indicating possibly increased intracranial pressure by associating increased stiffness with increased intracranial pressure.

16. A method as recited in clause 14 or 15, comprising detecting displacements as they vary with time at two locations around the optic nerve sheath or in the region surrounding the optic nerve sheath, wherein determining the measure of stiffness of the optic nerve sheath includes obtaining a parameter of deformability ($\Delta$) based on the displacements.

17. A method as recited in clause 16, wherein the parameter of deformability ($\Delta$) is calculated according to the equation:

$$\Delta = \frac{|d_A - d_B|}{d_A + d_B}$$

wherein $d_A$ and $d_B$ represent the displacements at the two locations.

18. A method for automated quantification of papilledema, the method comprising obtaining images of the eye circle and the optic nerve sheath after alignment of an ultrasound image obtained with an ultrasound probe with the principal direction in accordance with the method of any of clauses 1 to 13; processing of the images to identify the curve of the eye circle; and determining a measurement of papilledema with reference to the shape and size of formations at the back of the eye.

19. A method as recited in clause 18, comprising: using computer implemented image processing techniques to identify the curve of the eye circle; based on the intersection of the principal direction and the curve of the eye circle, identifying an area of the eye circle relevant to papilledema; and, using an image processing algorithm, finding one or more parameter(s) relating to the shape and/or size of a formation at the back of the eye in the area of the eye circle known to exhibit papilledema to thereby quantify the papilledema.

20. A computer programme product comprising instructions that, when executed, will configure a computer system including an ultrasound imaging device to carry out the method of any preceding clause.

21. A computer programme product as recited in clause 20, wherein the instructions will configure the computer system to:

use an ultrasound probe of the ultrasound imaging device to obtain images of anatomical structures in the region of the eye and across a region of interest expected to include the optic nerve;
process the images of said region of interest via computer implemented image processing techniques in order to identify boundary features in the images that are representative of the boundaries of at least one of the optic nerve and the optic nerve sheath;
use the identified boundary features of the imaged structure to determine a principal direction extending along the length of the optic nerve and the optic nerve sheath;
identify at least first and second points on the identified boundary features, wherein the first and second points are at different locations along the principal direction; and
guide the user to move the probe or control the probe to move the image plane of the probe in order to rotate the image plane of the probe until the first and second points are aligned in the image, and thereby guide the image plane of the probe to a required orientation for alignment of the ultrasound image with the principal direction.

22. A computer programme product as recited in clause 20 or 21, wherein the instructions will configure the computer system to:

use an ultrasound probe of the ultrasound imaging device to obtain images of a region including the eye circle and optic nerve;

using computer implemented image processing techniques, identify a principal direction of the optic nerve and the curve of the eye circle; based on the intersection of the principal direction and the curve of the eye circle, identify an area of the eye circle relevant to papilledema; and using an image processing algorithm, find one or more parameter(s) relating to the shape and/or size of a formation at the back of the eye in the area of the eye circle known to exhibit papilledema to thereby quantify the papilledema.

23. An apparatus for alignment of an ultrasound image obtained with an ultrasound probe for obtaining images in relation to the eye, the apparatus comprising:

an ultrasound imaging device including the ultrasound probe; and
a computer system configured to carry out the method of any of clauses 1 to 19.

24. An apparatus as recited in clause 23, wherein the computer system is configured to:

use the ultrasound probe of the ultrasound imaging device to obtain images of anatomical structures in the region of the eye and across a region of interest expected to include the optic nerve;
process the images of said region of interest via computer implemented image processing techniques in order to identify boundary features in the images that are representative of the boundaries of at least one of the optic nerve and the optic nerve sheath;
use the identified boundary features of the imaged structure to determine a principal direction extending along the length of the optic nerve and the optic nerve sheath;
identify at least first and second points of the identified boundary features, wherein the first and second points are at different locations along the principal direction; and
guide the user to move the probe or control the probe to move the image plane of the probe in order to rotate the image plane of the probe until the first and second points are aligned in the image, and thereby guide the probe to a required orientation for alignment of the ultrasound image with the principal direction.

25. An apparatus as recited in clause 23 or 24, wherein the computer system is configured to:

using computer implemented image processing techniques, identify a principal direction of the optic nerve and the curve of the eye circle; based on the intersection of the principal direction and the curve of the eye circle, identify an area of the eye circle relevant to papilledema; and using an image processing algorithm, find one or more parameter(s) relating to the shape and/or size of a formation at the back of the eye in the area of the eye circle known to exhibit papilledema to thereby quantify the papilledema.

**Claims**

1. An apparatus for automated quantification of papilledema, the apparatus comprising:

an ultrasound imaging device including an ultrasound probe; and
a computer system configured to:

(i) use the ultrasound probe of the ultrasound imaging device to obtain images of a region including the eye circle and optic nerve;
(ii) using computer implemented image processing techniques, identify a principal direction of the optic nerve and the curve of the eye circle;
(iii) based on the intersection of the principal direction and the curve of the eye circle, identify an area of the eye circle relevant to papilledema; and
(iv) using an image processing algorithm, find one or more parameter(s) relating to the shape and/or size of a formation at the back of the eye in the area of the eye circle known to exhibit papilledema, and thereby quantify the papilledema.

2. An apparatus as claimed in claim 1, wherein the computer system is configured to: identify a boundary of the optic nerve sheath, calculate the horizontal position and orientation of the optic nerve, calculate coordinates along the two sides of the optic nerve sheath, fit a line to the co-ordinates to find the direction along each of the two sides, and find an average of the two directions to thereby find the principal direction of the optic nerve sheath, optionally wherein the computer system is configured to calculate a principal eigenvector for each side during the step of fitting a line to find the direction along each of the two sides.

3. An apparatus as claimed in claim 2, comprising a neural network configured for identification of the boundary of the optic nerve sheath.

4. An apparatus as claimed in claim 2 or 3, wherein the one or more parameter(s) include one or more of: the maximum extent that a possible papilledema protrudes from the eye circle; an area of a possible papilledema; a volume of a possible papilledema, and/or an extent of a possible papilledema along the circumference of the eye circle.

5. An apparatus as claimed in claim 4, wherein the parameter includes the maximum extent that a possible papilledema protrudes from the eye circle and the computer system is configured to, for multiple points along the eye circle in the identified area of step (iii), determine the location of an inner edge of the possible papilledema and find the distance between the back of the eye circle and the inner edge of the possible papilledema, optionally wherein the computer system is configured to determine the location of the inner edge of the possible papilledema by using an edge detection method, such as via a step edge model.

6. An apparatus as claimed in claim 5, wherein the computer system is configured to, for the multiple points along the eye circle in the identified area of step (iii), sample the image intensity along a line starting from the outer edge of the eye circle and extending inwardly in the normal direction of the eye circle, and thereby determine the extent of the possible papilledema along each of multiple such lines, optionally wherein the line extending inwardly from the outer edge of the eye circle has a length in the range 1 mm to 4 mm.

7. An apparatus as claimed in claim 6, wherein the computer system is configured to determine the maximum extent of the possible papilledema along any one of the multiple lines and use that maximum extent as the parameter enabling quantification of the papilledema.

8. An apparatus as claimed in claim 1, wherein after identification of the principal direction in step (ii) the computer system is configured to:

identify at least first and second points on the identified boundary features, wherein the first and second points are at different locations along the principal direction; and rotate the image plane of the probe until the first and second points are aligned, and thereby determine a required orientation for alignment of the ultrasound image with the principal direction; wherein the computer system is configured to obtain one or more further ultrasound images with the probe in the required orientation for alignment with the principal direction, and wherein the computer system is configured to use these further ultrasound images to identify the area of the eye circle and to quantify the papilledema, optionally wherein the ultrasound probe includes an actuation mechanism for rotating the probe; and/or wherein the computer system is configured to use electronically controlled activations of the transducer array elements to steer the image plane to align the first and second points.

9. A computer implemented method for automated quantification of papilledema, the method comprising:

(i) obtaining ultrasound images of a region of interest including the eye circle and optic nerve; (ii) using computer implemented image processing techniques, identifying a principal direction of the optic nerve and the curve of the eye circle; (iii) based on the intersection of the principal direction and the curve of the eye circle, identifying an area of the eye circle relevant to papilledema; and (iv) using an image processing algorithm, finding one or more parameter(s) relating to the shape and/or size of a formation at the back of the eye in the area of the eye circle known to exhibit papilledema, and thereby quantifying the papilledema.

10. A computer implemented method as claimed in claim 9, wherein after obtaining the images the method is performed by computer without any human interaction.

11. A computer implemented method as claimed in claim 9 or 10, wherein the step of identifying the curve of the eye circle includes the use of image processing to determine relevant features of the ultrasound images, optionally wherein the step of identifying the curve of the eye circle includes segmentation of one or more of the ultrasound image(s) using a neural network.

12. A computer implemented method as claimed in any of claims 9 to 11, including calculating the position of the eye circle by determining point coordinates of the bottom of the eye and fitting a circle or partial circle to those point coordinates.

13. A computer implemented method as claimed in any of claims 9 to 12, wherein step (iii) includes identifying an area centred on the point of intersection and including points within a certain distance of the intersection of the principal direction and the eye circle, wherein this certain distance is measured circumferentially along the extent of the eye circle, optionally wherein the certain distance is in the range 1 mm to 5 mm to either side of the intersection point.

14. A computer implemented method as claimed in any of claims 9 to 13, wherein the method is performed by the apparatus of any of claims 1 to 8.

15. A computer programme product comprising instructions that, when executed, will configure a computer system including an ultrasound imaging device and/or the ability to import ultrasound image data from a storage device to carry out the method of any of claims 9 to 13,
optionally wherein the instructions will configure the computer system to use an ultrasound probe of the ultrasound imaging device to obtain images of a region of interest including the eye circle and optic nerve; using computer implemented image processing techniques, identify a principal direction of the optic nerve and the curve of the eye circle; based on the intersection of the principal direction and the curve of the eye circle, identify an area of the eye circle relevant to papilledema; and, using an image processing algorithm, find one or more parameter(s) relating to the shape and/or size of a formation at the back of the eye in the area of the eye circle known to exhibit papilledema, and thereby quantify the papilledema.

Fig. 1

Fig. 2

1

4

8

9

5

6

10

2

## Fig. 3

12

1

4

11

3

5

6

14

A

13

10

15

## Fig. 4

Fig. 5

Fig. 6

Fig. 7

Fig. 8

**REFERENCES CITED IN THE DESCRIPTION**

**Patent documents cited in the description**

- WO 2016193168 A

- US 8672851 B